# EUROPEAN PATENT APPLICATION

(11) **EP 1 798 555 A1**
(43) Date of publication of application: **20.06.2007**
(21) Application number: 05785751.8
(22) Date of filing: 21.09.2005
(51) Int. Cl.: G01N 33/53, G01N 21/78, G01N 33/15, G01N 33/50, G01N 37/00

(54) **METHOD OF DETECTING STRUCTURAL CHANGE OF PROTEIN IMMOBILIZED ON SUBSTRATE**

(30) Priority: 22.09.2004 JP 2004275013
(71) Applicant: Fuence Co., Ltd., Tokyo 150-0012 (JP)
(72) Inventor: NONAKA, Hiromi, FUENCE CO., LTD., Tokyo 1500012 (JP); KASE, Hiroshi, FUENCE CO., LTD., Tokyo 1500012 (JP); INOUE, Kozo, FUENCE CO., LTD., Tokyo 1500012 (JP)
(74) Representative: HOFFMANN EITLE
(86) International application number: PCT/JP2005/017394
(87) International publication number: WO 2006/033356

(57) **Abstract**

This invention provides a method for detection of conformational change of a protein immobilized on a substrate. In concrete, the method comprises, forming a sample film comprising a protein immobilized on a substrate, adding a substance to be detected for its activity to affect to the conformational change of the protein onto the sample film, and detecting the conformational change of the protein. The method enables to measure conformational change of a protein with immobilized state, using minute amount of protein in a short period for a number of samples in a short period all at once, rapidly and simply.

## Description

### BACKGROUND OF THE INVENTION

### 1. Field of the Invention

This invention relates to a method for detection of conformational change of a protein immobilized on a substrate. Furthermore, this invention relates to a method for screening a substance having an activity that affects to a conformational change of a protein, and a method for screening a therapeutic product or diagnostic product for a disease caused by a conformational change of a protein.

### 2. Related Art

As a means for detection of conformational change of a protein, techniques to investigate conformational change of a protein in a liquid phase have been known. For examples of such techniques, a method by reacting a protein with a dye that recognizes a particular structure of the protein and detecting the change in the amount of binding; FRET (Fluorescence Resonance Energy Transfer) method (Heyduk T., Curr.Opin.Biotech., Vol.13 p292-296 (2002)) comprising conformational analysis of a protein by energy transfer of two fluorescence molecules; Circular dichroism spectrometric method (K. Ogasawara, K. Aburaya, Protein, Nucleic acid, Enzyme Vol.49, p1668-1675 (2004)) that enables assumption of the contents of α-helix or β-sheet structure; Raman spectrometric method (T. Uchida, T. Kitagawa, Protein, Nucleic acid, Enzyme Vol.49, p1693-1699 (2004)) that enables assumption of protein folding; can be listed. As another technique, a method of conducting crystal analysis using electric microscopy is also known.

### SUMMARY OF THE INVENTION

However, as to the technique of conducting reaction in a solid phase, it is disadvantageous in that a large amount of protein is required for using a solid phase, moreover, labeling of a protein is needed in some occasions. In addition, a technique using microscopy has a problem that a large number of samples can not be observed all at once. Therefore, a method, that enables detection of conformational change of large number of proteins with a minute amount and simply, has been required.

To solve above-mentioned problem, this invention provides a method for detection of conformational change of a protein immobilized on a substrate. That is, this invention provides above-mentioned method comprising: forming a sample film comprising a protein immobilized on a substrate, adding a substance to be detected for its activity to affect to the conformational change of the protein onto the sample film, and detecting the conformational change of the protein. By adopting the method according to present invention using a protein immobilized onto a substrate, it comes to be possible to measure conformational change of a protein, using minute amount of protein in a short period for a number of samples all at once and rapidly.
Moreover, this invention also enables screening of ligands that affect to structural change of a protein efficiently. The most prominent feature of this invention lies in that present invention enabled detection of structural change of a protein with immobilized state, not using a reaction in a liquid phase like conventional techniques.

By using the method of present invention, a substance to be detected for its activity is added onto a sample film comprising an amyloid protein immobilized on a substrate, which is succeeded by conducting a reaction with a fluorescent dye that binds to β-sheet structure, and detecting the fluorescence intensity, thereby detection of the structural change of the substance can be achieved simply and rapidly in a short period. It is assumed that the method of the invention can be applied for detection of conformational changes of various proteins, not limited to amyloid protein.

### BRIEF DESCRIPTION OF THE DRAWINGS

[Fig.1] Fig.1 is a figure showing the structure of the micro flow channel chip.
[Fig.2] Fig.2 is a figure showing the aspect of immobilized bio-macromolecular at a shape of linear strip.
[Fig.3] FIG.3 is a graph showing the effect of ZnCl₂ on the structural change of amyloid β (1-42) to β-sheet structure.

### DESCRIPTION OF THE NOTES

1 First substrate
2 Spot
3 Second substrate
4 Concave portion
5 Inlet
6 Outlet

### BEST MODE FOR CARRYING OUT THE INVENTION

As described above, present invention provides a method for detection of conformational change of a protein immobilized on a substrate. Meanwhile, in this specification, "immobilization" means that a spot or a film is formed on a substrate from a sample dispersed or dissolved into a solvent at a stable condition, that is, at dried condition that maintained its biological or functional activity. As shown in the following examples, the inventors produced a sample film comprising amyloid protein immobilized on a substrate, and conformational change with addition of a substance to the sample film was detected. Meanwhile, though detection of conformational change of an amyloid protein is a preferred embodiment of this invention, however, the target protein to be detected for its conformational change is not limited to the conformational change of an amyloid protein.

Moreover, "conformational change" in this specification refers to a change in secondary or tertiary structure, such that conformation of a protein occurs. Even though a protein maintains the same primary structure, the function of the protein changes remarkably by change in its conformation, and conformational change of a protein is responsible for many important physiological functions. Therefore, detection of such conformational change in a protein has significant meaning in the field of experimental medicine as well as clinical medicine. In this specification, "amyloidgenic conformational change" means a conformational change that forms an amyloid fibril comprising a characteristic and layered β-sheet structure, and the amyloidgenic conformational change of a protein causes amyloid-related diseases. It is known that diseases such as Alzheimer disease are known to be caused by deposition of a protein having amyloidgenic conformational change. Therefore, the method of this invention that achieves efficient detection of conformational change at an immobilized condition would provide a new way for research or development of medicines for amyloid disease

In the examples of this specification, the inventors noticed on the conformational change of amyloid β (1-42), and detected conformational change of the protein using a fluorescence reagent that binds to conformational changed β-sheet structure. As the result, screening of a substance having the activity that affects to protein conformational change was enabled. However, not only amyloid P (1-42) used in the Examples, conformational change of various other amyloid β proteins, that is, amyloid P (1-40), amyloid β (11-40), amyloid P (11-42), N-terminal truncated amyloid P peptide, can be also detected by the same technique.

The proteins known to arise an amyloidgenic conformational change may include, but not limited to, amyloid P protein, immunoglobulin light chain protein, amyloid A protein, transthyretin protein, lysozyme, BriL protein, cystatin C protein, scrapie protein, β2 microglobulin, apolipoprotein A1, gelsolin, pancreatic islet amyloid protein, fibrinogen, prolactin, insulin, calcitonin, atrial natriuretic peptide, α-synuclein, prion protein, huntingtin protein, superoxide dismutase and α1-antichymotrypsin. Among all these, amyloid P protein is well-known as a typical protein that arises amyloidgenicconformational change, and detection of conformational change of amyloid β protein is a preferred embodiment according to this invention.

Meanwhile, according to present invention, conformational change of a protein is measured on a protein immobilized onto a solid phase. Therefore, at first, a sample film comprising a protein described above may be prepared on a substrate and used as a protein sample to be measured. The size of the sample film may preferably be 50 to 1000 µm in length, 200 to 2000 µm in width, and 0.3 to 10 µm in thickness, but not limited to these dimensions. Also, the substrate in the present invention may be an appropriate film support, which enables to carry the sample film to a measuring apparatus, by preparing the sample film on the support. The material and size of the substrate is not particularly limited. Meanwhile, as described below, the embodiment of preparing a sample film on the micro flow channel chip can be also adopted. Then according to such embodiment, the present invention can be achieved using a sample film much smaller than that described above.

As a means to prepare such sample film of protein, ESD method (electrospray deposition method), which forms a thin film by depositing the sample by electrospray method is preferred. The technique is known among those skilled in the art, so they can use such techniques for the purpose of this invention with proper modification. As an example of such reference disclosing such a technique, WO 2002-511792 can be listed, which describes a method of producing a deposition of nonvolatile substances including macro-biomolecules using electrospray.

According to ESD method, a protein can be immobilized moderately in loose and in porous. According to present invention, a protein is immobilized onto a substrate, however, if the immobilization is too tight, the conformational change of a protein is not allowed when a test substance is subjected to the protein, therefore it is not preferred in view of the purpose of this invention. However, if the immobilization of the protein is too loose, it may cause detachment of the protein when reaction occurs with various substances. From such aspect, the intensity of protein immobilization should be proper degree in strength, the ESD method is a technique that can satisfy such request. However, the method of protein immobilization is not limited to the ESD method, the method of spotting, and ink jet method (a method of film preparation by ink jet process) can be also utilized. Moreover, Japanese patent publication No. 2003-136005 discloses a device for preparing thin films or spots immobilized with bio-macromolecules while retaining their activities.

After immobilized by the ESD method, the protein constituting said sample film can be further cross-linked. Such cross-linking is not requisite, but it is effective for the purpose to maintain the shape and strength of the sample film as a film. Cross-linking reagents available for polymerizing biomolecules are well-known to those skilled in the art. For instance, Hermanson et al., Immobilized Affinity Ligand Techniques Academic Press, New York, 1991 can be used as a reference.

As a reagent used for cross-linking protein, glutaraldehyde, used in the following examples, is the most preferred. Moreover, the reagents for protein cross-linking may include, but are not limited to, zero-length cross-linking reagents such as 1-ethyl-3-(3-dimethylamino) propyl carbodiimide (EDC); homo-bifunctional cross-linking reagents such as dimethyl adipinimidate (DMA); hetero-bifunctional cross linking reagents such as succinimidyl 3-(2-pyridyldithio)propionate (SPDP); and trifunctional cross-linking reagents such as 4-azide-2-nitrophenylbiocytin-4-nitrophenyl ester. Further, the time period for cross-linking reaction is not specifically limited. The time period for cross-linking glutaraldehyde used in the following examples is five minutes, but the optimum condition may be selected accordingly within the range of about 0 to 3 hours.

After preparing a sample film comprising amyloid protein, which is immobilized onto a substrate, the sample film may be immersed into a buffer solution containing a test substance to be tested for its activity to cause conformational change of the protein. The substances which can be used as the test sample may include, but are not limited to, protein, peptide, amino acid, sugar, lipid, nucleic acid, metal and organic compound, and the effect of various test substances to cause conformational change of a protein can be investigated.

After subjecting the test substance to the protein constituting the sample film, it is reacted with a reagent that enables detection of conformational change of the protein. Such reagent is not particularly limited so far as the reagent can detect conformational change of the protein of the purpose, it may preferably be a fluorescent dye or an antibody. In the following Examples, the sample film is reacted with 1-fluoro-2,5-bis(3-carboxy-4-hyrdoxystyryl)benzene, which is a fluorescent dye that recognizes β-sheet structure, and the fluorescent intensity of the sample film is measured to evaluate the effect to the conformational change of an amyloid protein. However, there are some other reagents known to recognize β-sheet structure, for example, and reagents such as congo red, Crysamine-G, thioflavine T, BSB[(E,E)-1-bromo2,5-bis-(3-hydroxycarbonyl-4-hydroxy)styryl-benzene] are reported. Moreover, some derivatives of these compounds have similar effect. Therefore, a skilled artisan can select a reagent available for detection of β-sheet structure accordingly to be used for the purpose of this invention.

Meanwhile, the inventors developed a micro flow channel chip for the purpose to provide a biomolecule microchip, which has a structure that enables to detect binding of various proteins or DNAs to other compounds on the microchip, to harvest the bound compounds, and to identify them. It was reported in Japanese application No. 2002-243734. Therefore, in this invention, it is preferable to prepare a sample film of the protein of the target, to the micro flow channel chip described in Japanese application No. 2002-243734. In this specification, a micro flow channel chip means that described in Japanese application No. 2002-243734 or an altered micro flow channel chip according to the sample to be tested and the experimental conditions as needed. The micro flow channel chip used in this invention, however, should not be understood to be limited to that described in Japanese application No. 2002-243734, and other microchips can be also used within the spirit of the invention. In this specification, "a micro flow channel chip immobilized with a sample film" means a micro flow channel chip having a sample film immobilized with a protein to be detected on its conformational change.

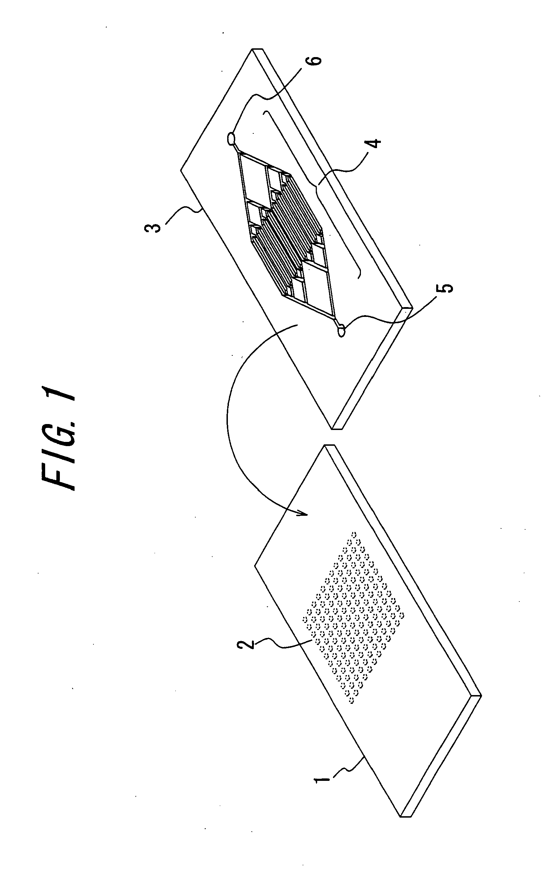

The micro flow channel chip described in Japanese application No. 2002-243734 is composed of spots of immobilized biomolecules, a substrate part that supports the spots, a minute flow channel part that supplies fluid, and a minute flow channel part for collecting the reactants. Therefore, using the micro flow channel chip described in Japanese application No. 2002-243734, binding between minute amount of biomolecule and the sample can be detected on the microchip, and the bound compound can be harvested for identified. Fig.1 shows the structure of the micro flow channel chip described in Japanese application No. 2002-243734.

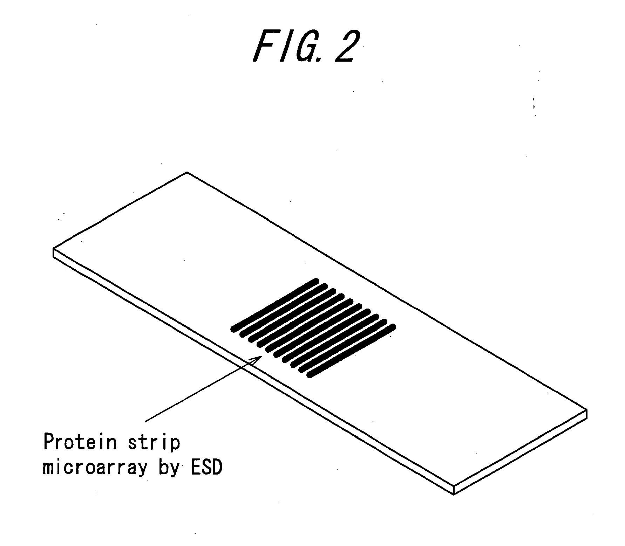

In the micro flow channel chip described in Japanese application No. 2002-243734 (Fig.1), an array of biomolecules spots 2 (in the case of present invention, a protein to be detected for its conformational change) are formed on the first substrate 1 made of glass or plastics. When biomolecules are immobilized on the substrate 1, the spots may be arranged to an array (Fig.1). Otherwise, a straight or curved strip, or one having an arbitrary shape may be used instead of the spots. Such spots or strip may be formed to have an arbitrary angle and an arbitrary position toward the micro flow channel, by forming deposition using electrospray deposition method in accordance to the purpose of usage. Meanwhile, a figure showing the aspect of immobilized bio-macromolecular at a shape of linear strip is shown in Fig.2. The micro flow channel chip described in Japanese application No. 2002-243734 further has a second substrate 3, and the second substrate 3 has a concave portion 4 in one surface thereof. The one surface, having the concave potion 4 of the second substrate 3 is bonded to a surface, having spots 2, of the first substrate 1. Owing to the bonding, closed micro flow channels and reaction regions are built between the substrates or in a gap therebetween. Liquid to react with is then to be properly supplied to them.

Both ends of the concave potion 4 of the second substrate 3 have through holes respectively, which holes are used as an inlet 5 for supplying liquid and an outlet 6 for recovering the liquid, respectively. The microchip is designed such that the liquid poured into the inlet 5 is supplied to the micro supply flow channels, in which one flow channel is diverged into a number of channels, to uniformly be fed to all spots in parallel. In addition in the microchip, after the branched liquid passes through the spots it would be collected into one flow recovery channel along with confluence of the channels to be recovered from the outlet 6. By using a micro flow channel chip having such structure, a sample substance can be flew through the minute flow channel of the micro flow channel chip to conduct reaction with a protein, then a reagent for detection can be flew through for detection of the conformational change of the protein, thereby minute conformational change of the protein can be detected with high sensitivity and accuracy in a short period.

In concrete, a sample film comprising a protein is formed at the portion corresponding spot 2 of Fig.1 by ESD method. Then, the first substrate 1 on which spot 2 is formed is bonded with the second substrate 3 equipped with concave portion 4. By flowing the test substance dissolved in a solvent through the minute channel, the spot 2 serves as a reaction position of the protein and the test substance, then the conformational change of the protein in the sample film occurs. Thereafter, a fluorescent reagent or an antibody that specifically recognizes a protein which received conformational change is flew through the minute channels, thereby conformational change of the protein can be detected on the micro flow channel chip. In this specification, "a reaction position on the minute channel" means a position on which a sample film is formed on the first substrate 1, and provides a position for reaction with the liquid sample flowing through the minute channel when combined with the second substrate 3. The method to have the immobilized protein contacted with the test substance is not limited to the embodiment using the micro flow channel chip, the embodiment using the micro flow channel chip is especially preferable in this invention, for only minute sample is needed in such embodiment. Meanwhile, the shape of "substrate" to be used to immobilize the protein is not particularly limited, any shape can be adopted according to the purpose of usage. In concrete, in addition to the minute flow channels definitely described above, a micro-well can be also adopted.

By the way, detection of amyloidgenic conformational change of a protein has been described mainly so far, the conformational change detected by this invention is not limited to such embodiment. As to example of other conformational change for which the method of this invention is assumed to be available, dimerization of growth hormone receptor can be mentioned. Dimerization of growth hormone occurs upon stimulation by GH, a ligand for GH receptor, and it is assumed that signal of GH stimulation is intracellular transduced through dimerization of growth hormone receptor upon GH stimulation. An antibody is known and the antibody recognizes GH receptor epitope having sensitivity to such GH receptor dimerization and binds to the dimerized GH receptor (Yue Zhang et al., J.Biol.Chem., Vol.274, pp33072-33084 (1999)). Then a sample film of GH receptor can be prepared and binding activity of the antibody labeled with fluorescent can be detected, then dimerization of GH receptor immobilized on a substrate can be detected.

In the case of a receptor that dimerizes upon ligand stimulation such as that represented by GH receptor, an antagonist or an agonist of the receptor can be screened by detection of conformational change of dimerization. In addition to GH receptor, some cytokine receptors such as CSF-1 (colony stimulating factor-1) receptor or PDGF (platelet derived growth factor) receptor and G protein coupled-type receptors also form dimers upon ligand stimulation. Moreover, as to these receptors, agonists or antagonists of the receptors can be also screened in the same manner.

Thus, a substance that effects to conformational change of a protein can be screened by the method for detection of conformational change of a protein described above. According to the method of this invention that detects conformational change of a protein immobilized on a substrate, a substance that affects to conformational change of a protein can be selected efficiently, and the time or the amount of protein needed for screening can be reduced significantly.

Moreover, an active substance obtained by such screening can be a therapeutic product or diagnostic product for a disease caused by conformational change of a protein. For example, conformational change of an amyloid protein to β-sheet structure causes Alzheimer disease. Therefore, a substance can be a therapeutic product of Alzheimer disease, if the substance inhibits the change under a condition where conformational change of an amyloid protein is likely to occur. As described above, the method of this invention has various possibilities on the fields of experimental medicine and clinical diagnosis.

### EXAMPLES

### Effect of ZnCl₂ on the change of amyloid β immobilized on a substrate

Amyloid β (1-42) (Bachem AG, Budendorf, Switzerland) was dissolved in 0.1% ammonia water at the concentration of 1mg/mL. This solution was sprayed under dry air using an electrospry device described in WO 2002-511792 or an immobilzing device described in Japanese Patent Publication No.2003-136005. The solution was permeated through a mask with holes of 400 µm in length and 800 µm in width, and then a film having 1 µm thickness was prepared using an electrospray method (the EDS method), then protein was cross-linked at 30 °C for 5 minutes by glutaraldehyde.

The resulting film was immersed into 10 mM Hepes pH7.4 buffer solution (hereinafter referred to as "the buffer solution") containing 0.15 M NaCl for 10 minutes, in the presence or absence of ZnCl₂, except that the EDTA sample was only reacted with 1 mM ZnCl₂ and then immersed in a buffer solution containing 1 mM EDTA for 10 minutes. After that the film was immersed into a solution of 1-fluoro-2,5-bis(3-carboxy-4-hyrdoxystyryl)benzene (Dojindo Chemical Laboratory Co., Kumamoto) that binds to β-sheet structure, prepared to the final concentration of 0.01% using 50% ethanol solution. Furthermore, the film was immersed into a solution of saturated lithium carbonate, then it was lightly washed by 50% ethanol, then dried and observed under fluorescent microscope.

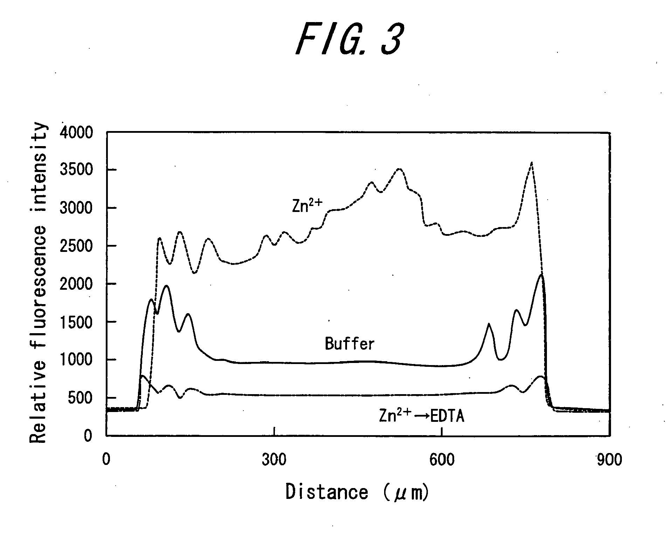

As to the film reacted under the presence of ZnCl₂ (Fig. 3 : dotted line), the film reacted with only the buffer solution in the absence of ZnCl₂ (Fig.3 : solid line), the film reacted with EDTA (replaced with EDTA after reaction with ZnCl₂) (Fig.3 : dashed line), the relative intensities were obtained by scanning relative intensities on each films (Fig.3). Meanwhile, in Fig.3, the vertical axis represents the relative intensity and the horizontal axis represents the distance from the left periphery of the film when scanning was conducted on the film formed by protein. The portions where the values are higher than the baseline correspond to the portions where the film exists. The fluorescent intensity of the film immersed into 1 mM ZnCl₂ was shown to be the highest, indicating that amyloid β changed to its β sheet structure.

### (Industrial applicability)

A substance to be tested for its activity to cause conformational change can be added to a sample film comprising a protein immobilized onto a substrate, then a reagent that enables detection of the conformational change can be subjected to the film, thereby the conformational change of the protein caused by said substance can be detected. According to this invention, for the protein is immobilized on the substrate, conformational change of the protein can be detected simply and rapidly in a short period. It is assumed that the method of the invention can be applied to screening of substances that affect to conformational change of a protein, furthermore, it provides a new way for development of therapeutic products or diagnostic products for diseases caused by conformational change of a protein.

## Claims

1. A method for detection of conformational change of a protein immobilized on a substrate, the method comprising:
forming a sample film comprising a protein immobilized on a substrate,
adding a substance to be detected for its activity to affect to the conformational change of the protein onto the sample film, and
detecting the conformational change of the protein.

2. The method according to Claim 1, wherein said conformational change is a conformational change to amyloidal β-sheet structure.

3. The method according to Claim 1, wherein said protein is selected from the group consisting of amyloid β protein, immunoglobulin light chain protein, amyloid A protein, transthyretin protein, lysozyme, BriL protein, cystatin C protein, scrapie protein, β2 microglobulin, apolipoprotein A1, gelsolin, pancreatic islet amyloid protein, fibrinogen, prolactin, insulin, calcitonin, atrial natriuretic peptide, α-synuclein, prion protein, huntingtin protein, superoxide dismutase and α1-antichymotrypsin.

4. The method according to Claim 3, wherein said protein is amyloid β protein.

5. The method according to any one of Claims 1 to 4, wherein said immobilization is conducted by electrospray deposition method.

6. The method according to any one of Claims 1 to 5, wherein said conformational change is detected by a fluorescent reagent that specifically recognizes a protein which received said conformational change, or an antibody that specifically recognizes a protein which received said conformational change.

7. A method for screening a substance having an activity that affects to conformational change of a protein by the method according to any one of Claims 1 to 6.

8. A method for screening a therapeutic product or a diagnostic product for a disease caused by conformational change of a protein by the method according to any one of Claims 1 to 6.

9. A micro flow channel chip immobilized with a sample film for detection of conformational change of a protein, the sample film comprising the protein immobilized at a reaction position on minute channel of the micro flow channel chip.

10. The micro flow channel chip immobilized with a sample film according to Claim 9, wherein said immobilization is conducted by electrospray deposition method.

11. The method according to Claim 9, wherein said conformational change is a conformational change to amyloidal β-sheet structure.

12. A method for detection of conformational change of a protein by the micro flow channel chip immobilized with a sample film according to any one of Claims 9 to 11.
